# EUROPEAN PATENT APPLICATION

(11) **EP 4 775 167 A1**
(43) Date of publication of application: **15.07.2026**
(21) Application number: 26150730.5
(22) Date of filing: 08.01.2026
(51) Int. Cl.: A61B 34/20

(54) **POSITIONING DEVIATION WARNING METHOD FOR SURGICAL NAVIGATION AND SYSTEM THEREOF**

(30) Priority: 10.01.2025 US 202563743647 P; 09.12.2025 TW 114148127
(71) Applicant: Remex Medical Corp., Taichung City 40852 (TW)
(72) Inventor: CHENG, Ying-Yi, Taichung City (TW); LIN, Chen-Tai, Taichung City (TW); LIN, Sheng-Fang, Taichung City (TW)
(74) Representative: Vossius & Partner Patentanwälte Rechtsanwälte mbB

(57) **Abstract**

A positioning deviation warning method for surgical navigation includes configuring a navigation device to monitor a plurality of reference coordinates of a reference; configuring the navigation device to calculate a plurality of first distance values between the reference and the navigation device according to the reference coordinates; configuring the navigation device to extract a portion of the first distance values according to a first time window and calculate a first amplitude of the portion of the first distance values within the first time window; extracting, according to a second time window corresponding to a time point, a portion of a plurality of the first amplitudes corresponding to the time points and calculate a second amplitude of the portion of the first amplitudes within the second time window; and determining whether the second amplitude of the portion of the first amplitudes is greater than a predetermined threshold.

## Description

### BACKGROUND

### Technical field

The present disclosure relates to a positioning deviation warning method and a system thereof, and more particularly to a positioning deviation warning method for surgical navigation and a system thereof.

### Description of Related Art

Generally, a reference, serving as a marker and positioning element for locating a patient or a surgical instrument, plays a key role in a surgical navigation system. However, during surgery, positioning deviation may occur due to the following main factors: First, human interference: medical personnel adjust or accidentally touch the surgical bed, causing positioning deviation of the reference. Second, physiological interference: chest and abdominal undulations caused by patient breathing, leading to periodic displacement of the reference.

Regarding the marking and positioning of the patient, its minute displacements are often difficult to perceive, where non-periodic accidental displacements are not easily discovered, and periodic displacements caused by breathing require differentiated handling; and the aforementioned two types of displacements necessitate distinct processing strategies. Current conventional techniques cannot achieve real-time monitoring and cannot effectively distinguish between non-periodic accidental displacements and periodic displacements caused by breathing.

Regarding the marking and positioning of surgical instruments, the position and angle of the surgical instruments are adjusted during the surgical process, and after adjustment is completed, it is necessary to wait until the instruments are stable and motionless before proceeding with imaging and positioning. Current conventional techniques lack objective stability judgment criteria during the adjustment of surgical instruments. This easily results in that users rely solely on experience to judge whether the surgical instrument is stable and cannot precisely grasp the optimal imaging timing, and positioning accuracy may be impaired due to premature or delayed imaging.

Furthermore, regarding the optical tracker of the navigation device, it possesses distance-related accuracy characteristics, including an optimal positioning range (e.g., the highest accuracy can be achieved within a specific distance), accuracy attenuation characteristics (e.g., positioning accuracy gradually decreases after exceeding a boundary distance), and distance sensitivity (e.g., positioning accuracy is inversely proportional to the operating distance). Current conventional techniques face difficulties during the operation of the navigation device, including the inability to grasp the current operating distance in real time, the lack of objective distance judgment criteria, and the difficulty in ensuring the positioning system is in an optimal working state.

In view of this, there is currently a lack in the market of a positioning deviation warning method for surgical navigation and a system thereof capable of detecting in real time, effectively distinguishing between non-periodic and periodic displacements, warning of abnormal displacements for deviation elimination, possessing objective judgment criteria, prompting the optimal imaging timing, providing an objective distance status, and ensuring that the positioning system is in an optimal working state. Therefore, relevant practitioners are seeking solutions.

### SUMMARY

According to one implementation of the method aspect of the present disclosure, a positioning deviation warning method for surgical navigation is provided, including: configuring a navigation device to monitor a plurality of reference coordinates of a reference; configuring the navigation device to calculate a plurality of first distance values between the reference and the navigation device according to the reference coordinates, wherein each of the first distance values is a distance between each of the reference coordinates and the navigation device; configuring the navigation device to extract a portion of the first distance values according to a first time window and calculate a first amplitude of the portion of the first distance values within the first time window, wherein the portion of the first distance values includes a plurality of second distance values, and the first amplitude is equal to a difference between a maximum distance value and a minimum distance value among the second distance values; configuring the navigation device to extract, according to a second time window corresponding to a time point, a portion of a plurality of the first amplitudes corresponding to a plurality of the time points and calculate a second amplitude of the portion of the first amplitudes within the second time window, wherein the portion of the first amplitudes includes a plurality of amplitude values, and the second amplitude is equal to a difference between a maximum amplitude value and a minimum amplitude value among the amplitude values; and configuring the navigation device to determine whether the second amplitude of the portion of the first amplitudes is greater than a predetermined threshold.

According to one embodiment, the navigation device determines whether the second amplitude is greater than the predetermined threshold to generate a determination result, and generates a warning message according to the determination result to inform a user that the reference has undergone positioning deviation.

According to one embodiment, the first time window is greater than the second time window.

According to one embodiment, the first time window is a window from the time point forward by a first time value; and the second time window is a window from the time point forward by a second time value.

According to one embodiment, the positioning deviation warning method for surgical navigation further includes configuring the navigation device to determine one of the first distance values according to a predetermined distance interval set to generate a distance determination result, and generate an optical signal according to the distance determination result. The predetermined distance interval set includes a first predetermined distance threshold and a second predetermined distance threshold, the first predetermined distance threshold is less than the second predetermined distance threshold, the optical signal has a color, and the color is one of a green, a yellow, and an orange.

According to one embodiment, when the distance determination result is that the one of the first distance values is less than the first predetermined distance threshold, the color of the optical signal is the green. When the distance determination result is that the one of the first distance values is greater than or equal to the first predetermined distance threshold and less than the second predetermined distance threshold, the color of the optical signal is the yellow. When the distance determination result is that the one of the first distance values is greater than the second predetermined distance threshold, the color of the optical signal is the orange.

According to one embodiment, the positioning deviation warning method for surgical navigation further includes configuring the navigation device to monitor a plurality of dynamic reference coordinates of a dynamic reference; configuring the navigation device to calculate a plurality of dynamic distance values between the dynamic reference and the navigation device according to the dynamic reference coordinates, wherein each of the dynamic distance values is a distance between each of the dynamic reference coordinates and the navigation device; configuring the navigation device to extract a portion of the dynamic distance values according to a time window and calculate a standard deviation of the portion of the dynamic distance values within the time window; and configuring the navigation device to determine whether the standard deviation of the portion of the dynamic distance values is greater than another predetermined threshold.

According to one embodiment, the reference is disposed on a first target object, the dynamic reference is disposed on a second target object, and the first target object is different from the second target object.

According to another implementation of the method aspect of the present disclosure, a positioning deviation warning method for surgical navigation is provided, including: configuring a navigation device to monitor a plurality of reference coordinates of a reference; configuring the navigation device to calculate a plurality of distance values between the reference and the navigation device according to the reference coordinates, wherein each of the distance values is a distance between each of the reference coordinates and the navigation device; configuring the navigation device to extract a portion of the distance values according to a time window and calculate a standard deviation of the portion of the distance values within the time window; and configuring the navigation device to determine whether the standard deviation of the portion of the distance values is greater than a predetermined standard deviation threshold.

According to one embodiment, the navigation device determines whether the standard deviation is greater than the predetermined standard deviation threshold to generate a determination result, and generates a warning message according to the determination result to inform a user that the reference has undergone positioning deviation.

According to one embodiment, the time window is a window from a time point forward by a time value.

According to one implementation of the structural aspect of the present disclosure, a positioning deviation warning system for surgical navigation is provided, including a reference and a navigation device. The navigation device is disposed toward the reference and configured to perform operations including: monitoring a plurality of reference coordinates of the reference; calculating a plurality of first distance values between the reference and the navigation device according to the reference coordinates, wherein each of the first distance values is a distance between each of the reference coordinates and the navigation device; extracting a portion of the first distance values according to a first time window and calculating a first amplitude of the portion of the first distance values within the first time window, wherein the portion of the first distance values included a plurality of second distance values, and the first amplitude is equal to a difference between a maximum distance value and a minimum distance value among the second distance values; extracting, according to a second time window corresponding to a time point, a portion of a plurality of the first amplitudes corresponding to a plurality of the time points and calculating a second amplitude of the portion of the first amplitudes within the second time window, wherein the portion of the first amplitudes includes a plurality of amplitude values, and the second amplitude is equal to a difference between a maximum amplitude value and a minimum amplitude value among the amplitude values; and determining whether the second amplitude of the portion of the first amplitudes is greater than a predetermined threshold.

According to one embodiment, the positioning deviation warning system for surgical navigation further includes a display device connected to the navigation device and configured to display a warning message. The navigation device determines whether the second amplitude is greater than the predetermined threshold to generate a determination result, and generates the warning message according to the determination result to inform a user that the reference has undergone positioning deviation.

According to one embodiment, the first time window is greater than the second time window.

According to one embodiment, the first time window is a window from the time point forward by a first time value; and the second time window is a window from the time point forward by a second time value.

According to one embodiment, the navigation device is configured to perform operations further include configuring the navigation device to determine one of the first distance values according to a predetermined distance interval set to generate a distance determination result, and generate an optical signal according to the distance determination result. The predetermined distance interval set includes a first predetermined distance threshold and a second predetermined distance threshold, the first predetermined distance threshold is less than the second predetermined distance threshold, the optical signal has a color, and the color is one of a green, a yellow, and an orange.

According to one embodiment, when the distance determination result is that the one of the first distance values is less than the first predetermined distance threshold, the color of the optical signal is the green. When the distance determination result is that the one of the first distance values is greater than or equal to the first predetermined distance threshold and less than the second predetermined distance threshold, the color of the optical signal is the yellow. When the distance determination result is that the one of the first distance values is greater than the second predetermined distance threshold, the color of the optical signal is the orange.

According to one embodiment, the reference is disposed on a first target object, and the positioning deviation warning system for surgical navigation further includes a dynamic reference disposed on a second target object. The first target object is different from the second target object. The navigation device is configured to perform operations further include monitoring a plurality of dynamic reference coordinates of a dynamic reference; calculating a plurality of dynamic distance values between the dynamic reference and the navigation device according to the dynamic reference coordinates, wherein each of the dynamic distance values is a distance between each of the dynamic reference coordinates and the navigation device; extracting a portion of the dynamic distance values according to a time window and calculating a standard deviation of the portion of the dynamic distance values within the time window; and determining whether the standard deviation of the portion of the dynamic distance values is greater than another predetermined threshold.

### BRIEF DESCRIPTION OF THE DRAWINGS

The present disclosure can be more fully understood by reading the following detailed description of the embodiment, with reference made to the accompanying drawings as follows:
FIG. 1 is a schematic diagram illustrating a positioning deviation warning system for surgical navigation according to a first embodiment of the present disclosure.
FIG. 2 is a flowchart illustrating a positioning deviation warning method for surgical navigation according to a second embodiment of the present disclosure.
FIG. 3 is a flowchart illustrating a positioning deviation warning method for surgical navigation according to a third embodiment of the present disclosure.
FIG. 4 is a waveform diagram illustrating motion detection for a reference according to the present disclosure.
FIG. 5 is a flowchart illustrating a positioning deviation warning method for surgical navigation according to a fourth embodiment of the present disclosure.
FIG. 6 is a flowchart illustrating a positioning deviation warning method for surgical navigation according to a fifth embodiment of the present disclosure.
FIG. 7 is a waveform diagram illustrating motion detection for a dynamic reference according to the present disclosure.
FIG. 8 is a flowchart illustrating a positioning deviation warning method for surgical navigation according to a sixth embodiment of the present disclosure.

### DETAILED DESCRIPTION

The embodiment will be described with the drawings. For clarity, some practical details will be described below. However, it should be noted that the present disclosure should not be limited by the practical details, that is, in some embodiment, the practical details is unnecessary. In addition, for simplifying the drawings, some conventional structures and elements will be simply illustrated, and repeated elements may be represented by the same labels.

It will be understood that when an element (or device) is referred to as be "connected to" another element, it can be directly connected to the other element, or it can be indirectly connected to the other element, that is, intervening elements may be present. In contrast, when an element is referred to as be "directly connected to" another element, there are no intervening elements present. In addition, the terms first, second, third, etc. are used herein to describe various elements or components, these elements or components should not be limited by these terms. Consequently, a first element or component discussed below could be termed a second element or component.

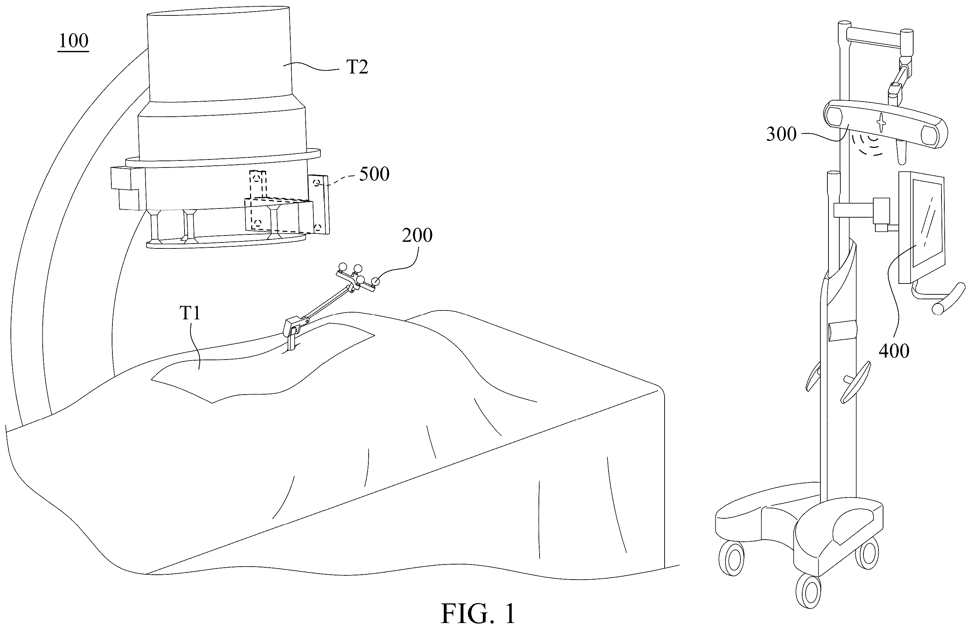

Reference is made to FIG. 1, which is a schematic diagram illustrating a positioning deviation warning system 100 for surgical navigation according to a first embodiment of the present disclosure. The positioning deviation warning system 100 for surgical navigation includes a reference 200 and a navigation device 300. The navigation device 300 is disposed toward the reference 200 and configured to perform operations including: monitoring a plurality of reference coordinates of the reference 200; calculating a plurality of first distance values between the reference 200 and the navigation device 300 according to the reference coordinates, where each of the first distance values is a distance between each of the reference coordinates and the navigation device 300; extracting a portion of the first distance values according to a first time window and calculating a first amplitude of the portion of the first distance values within the first time window, where the portion of the first distance values includes a plurality of second distance values, and the first amplitude is equal to a difference between a maximum distance value and a minimum distance value among the second distance values; extracting, according to a second time window corresponding to a time point, a portion of a plurality of the first amplitudes corresponding to a plurality of the time points and calculating a second amplitude of the portion of the first amplitudes within the second time window, where the portion of the first amplitudes includes a plurality of amplitude values, and the second amplitude is equal to a difference between a maximum amplitude value and a minimum amplitude value among the amplitude values; and determining whether the second amplitude of the portion of the first amplitudes is greater than a predetermined threshold.

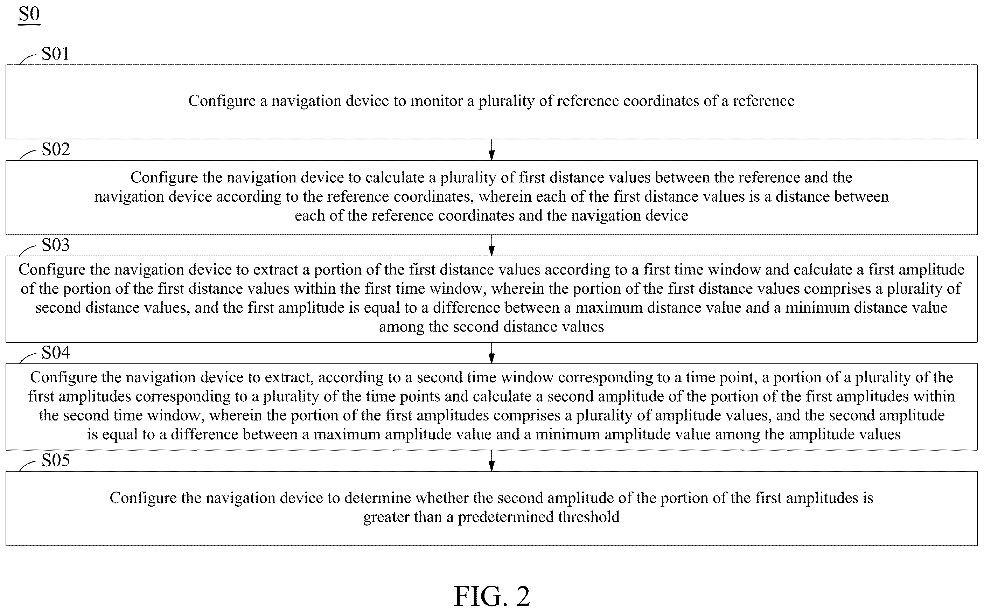

Reference is made to FIGS. 1 and 2 collectively, where FIG. 2 is a flowchart illustrating a positioning deviation warning method S0 for surgical navigation according to a second embodiment of the present disclosure. The positioning deviation warning method S0 for surgical navigation is applied to the positioning deviation warning system 100 for surgical navigation, and includes performing steps S01, S02, S03, S04, and S05. Step S01 includes configuring a navigation device 300 to monitor a plurality of reference coordinates of a reference 200. Step S02 includes configuring the navigation device 300 to calculate a plurality of first distance values between the reference 200 and the navigation device 300 according to the reference coordinates, where each of the first distance values is a distance between each of the reference coordinates and the navigation device 300. Step S03 includes configuring the navigation device 300 to extract a portion of the first distance values according to a first time window and calculate a first amplitude of the portion of the first distance values within the first time window, where the portion of the first distance values includes a plurality of second distance values, and the first amplitude is equal to a difference between a maximum distance value and a minimum distance value among the second distance values. Step S04 includes configuring the navigation device 300 to extract, according to a second time window corresponding to a time point, a portion of a plurality of the first amplitudes corresponding to a plurality of the time points and calculate a second amplitude of the portion of the first amplitudes within the second time window, where the portion of the first amplitudes includes a plurality of amplitude values, and the second amplitude is equal to a difference between a maximum amplitude value and a minimum amplitude value among the amplitude values. Step S05 includes configuring the navigation device 300 to determine whether the second amplitude of the portion of the first amplitudes is greater than a predetermined threshold.

Accordingly, in the positioning deviation warning system 100 for surgical navigation and the positioning deviation warning method S0 for surgical navigation according to the present disclosure, by determining the second amplitude of the first amplitudes of the distance values (i.e., an amplitude of amplitudes of the distance values) to confirm whether the reference 200 has undergone positioning deviation under the condition that the navigation device 300 does not undergo positioning deviation, not only can real-time detection be effectively achieved, but also non-periodic and periodic displacements can be effectively distinguished, thereby achieving the effect of positioning deviation warning.

In FIG. 1, the positioning deviation warning system 100 for surgical navigation may further includes a display device 400 and a dynamic reference 500, where the display device 400 is connected to the navigation device 300 and configured to display a warning message. The reference 200 is disposed on a first target object T1, the dynamic reference 500 is disposed on a second target object T2, and the first target object T1 is different from the second target object T2. Any one of the reference 200 and the dynamic reference 500 includes a plurality of reflective spheres, which can be regarded as markers. The navigation device 300 may include an optical tracker and a processor, the optical tracker being electrically connected to the processor. The optical tracker is configured to detect the reference 200 or/and the dynamic reference 500 to generate reference coordinates or/and dynamic reference coordinates. In one embodiment, the first target object T1 may be a patient (a site of the patient's body), and the second target object T2 may be a C-arm (a mobile X-ray machine). The processor receives coordinate data from the optical tracker and performs subsequent calculation and determination. The processor may be an artificial intelligence (AI) chip, a central processing unit (CPU), a graphics processing unit (GPU), or other high-speed processor, but the present disclosure is not limited thereto.

When the navigation device 300 detects the dynamic reference 500, the navigation device 300 is configured to perform operations further including: monitoring a plurality of dynamic reference coordinates of the dynamic reference 500; calculating a plurality of dynamic distance values between the dynamic reference 500 and the navigation device 300 according to the dynamic reference coordinates, where each of the dynamic distance values is a distance between each of the dynamic reference coordinates and the navigation device 300; extracting a portion of the dynamic distance values according to a time window and calculating a standard deviation of the portion of the dynamic distance values within the time window; and determining whether the standard deviation of the portion of the dynamic distance values is greater than another predetermined threshold.

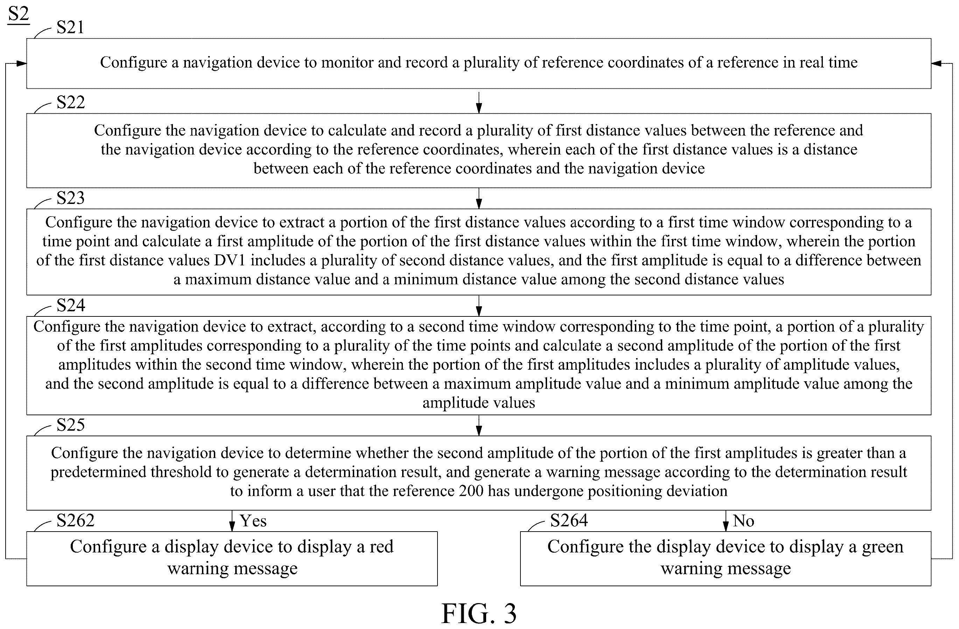

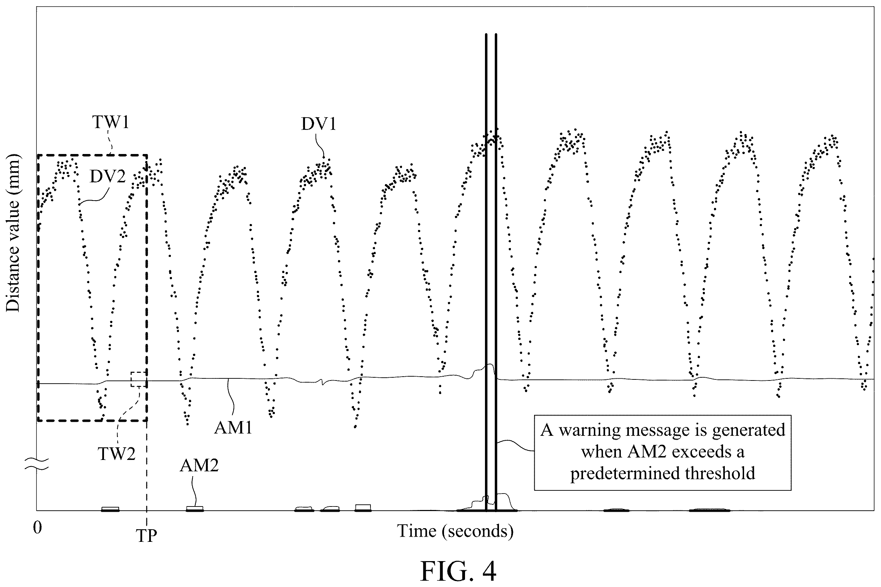

Reference is made to FIGS. 1, 3, and 4 collectively, where FIG. 3 is a flowchart illustrating a positioning deviation warning method S2 for surgical navigation according to a third embodiment of the present disclosure; and FIG. 4 is a waveform diagram illustrating motion detection for a reference 200 according to the present disclosure. The positioning deviation warning method S2 for surgical navigation in FIG. 3 is applied to the positioning deviation warning system 100 for surgical navigation, and includes performing steps S21, S22, S23, S24, S25, S262, and S264. In FIG. 4, the horizontal axis represents time (seconds), and the vertical axis represents distance value (mm).

Step S21 includes configuring a navigation device 300 to monitor and record a plurality of reference coordinates of a reference 200 in real time. Step S22 includes configuring the navigation device 300 to calculate and record a plurality of first distance values DV1 between the reference 200 and the navigation device 300 according to the reference coordinates, where each of the first distance values DV1 is a distance between each of the reference coordinates and the navigation device 300. Step S23 includes configuring the navigation device 300 to extract a portion of the first distance values DV1 according to a first time window TW1 corresponding to a time point TP and calculate a first amplitude AM1 of the portion of the first distance values DV1 within the first time window TW1, where the portion of the first distance values DV1 includes a plurality of second distance values DV2, and the first amplitude AM1 is equal to a difference between a maximum distance value and a minimum distance value among the second distance values DV2. Step S24 includes configuring the navigation device 300 to extract, according to a second time window TW2 corresponding to the time point TP, a portion of a plurality of the first amplitudes AM1 corresponding to a plurality of the time points TP and calculate a second amplitude AM2 of the portion of the first amplitudes AM1 within the second time window TW2, where the portion of the first amplitudes AM1 includes a plurality of amplitude values, and the second amplitude AM2 is equal to a difference between a maximum amplitude value and a minimum amplitude value among the amplitude values. Step S25 includes configuring the navigation device 300 to determine whether the second amplitude AM2 of the portion of the first amplitudes AM1 is greater than a predetermined threshold to generate a determination result, and generate a warning message according to the determination result to inform a user that the reference 200 has undergone positioning deviation. When the determination result is yes, step S262 is performed; and when the determination result is no, step S264 is performed. Step S262 includes configuring a display device 400 to display a red warning message (indicating that the reference 200 is unstable). Step S264 includes configuring the display device 400 to display a green warning message (indicating that the reference 200 is stable).

Specifically, the first time window TW1 is greater than the second time window TW2. The first time window TW1 is a window from the time point TP forward by a first time value, and the first time value may be in the range of several seconds to several tens of seconds, preferably greater than 5 seconds and less than 11 seconds. The second time window TW2 is a window from the time point TP forward by a second time value, and the second time value may be in the range of several hundred milliseconds to several seconds, preferably greater than 0 seconds and less than 2 seconds. The predetermined threshold may be greater than 0.10 mm and less than 0.20 mm. In one embodiment, the first time value may be 8 seconds, the second time value may be 1 second, and the predetermined threshold may be 0.15 mm, but the present disclosure is not limited thereto. In other embodiments, the warning message may be a sound which may be emitted by the navigation device 300 or the display device 400 to inform the user that the reference 200 has undergone positioning deviation.

Accordingly, in the positioning deviation warning method S2 for surgical navigation according to the present disclosure, by determining the second amplitude AM2 of the first amplitudes AM1 of the distance values (i.e., an amplitude of amplitudes of the distance values) to confirm whether the reference 200 has undergone positioning deviation under the condition that the navigation device 300 does not undergo positioning deviation, not only can real-time detection be effectively achieved, but also non-periodic and periodic displacements can be effectively distinguished. Furthermore, according to the present disclosure, a warning message of a specific color is utilized to allow the user to immediately learn of an abnormal displacement (non-periodic displacement), thereby achieving positioning deviation elimination and enhancing surgical accuracy and safety.

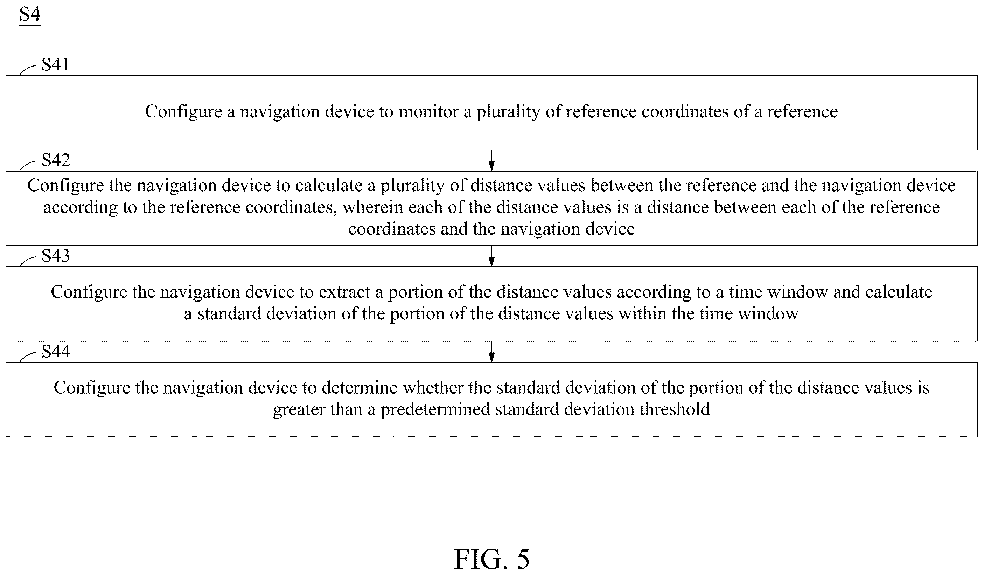

Reference is made to FIGS. 1 and 5 collectively, where FIG. 5 is a flowchart illustrating a positioning deviation warning method S4 for surgical navigation according to a fourth embodiment of the present disclosure. The positioning deviation warning method S4 for surgical navigation is applied to the positioning deviation warning system 100 for surgical navigation, and includes performing steps S41, S42, S43, S44. Step S41 includes configuring a navigation device 300 to monitor a plurality of reference coordinates of a reference, where the reference (reference module) may be a reference 200 disposed on a first target object T1 (e.g., a patient), or may be a dynamic reference 500 disposed on a second target object T2 (e.g., a C-arm), but the present disclosure is not limited thereto. Step S42 includes configuring the navigation device 300 to calculate a plurality of distance values between the reference and the navigation device 300 according to the reference coordinates, where each of the distance values is a distance between each of the reference coordinates and the navigation device 300. Step S43 includes configuring the navigation device 300 to extract a portion of the distance values according to a time window and calculate a standard deviation of the portion of the distance values within the time window. Step S44 includes configuring the navigation device 300 to determine whether the standard deviation of the portion of the distance values is greater than a predetermined standard deviation threshold.

Accordingly, in the positioning deviation warning method S4 for surgical navigation according to the present disclosure, by determining the standard deviation of the distance values to confirm whether the reference module has undergone positioning deviation under the condition that the navigation device 300 does not undergo positioning deviation, the degree of stability of the reference module can be displayed in real time, objective judgment criteria can be provided, and the optimal imaging timing can be prompted, thereby enhancing the image positioning accuracy, optimizing the surgical workflow efficiency, reducing the uncertainty of subjective judgment by the operator, and improving the overall system reliability. Furthermore, the present disclosure provides more precise and objective equipment stability monitoring for surgical navigation, ensuring that image capture and positioning are performed under optimal conditions.

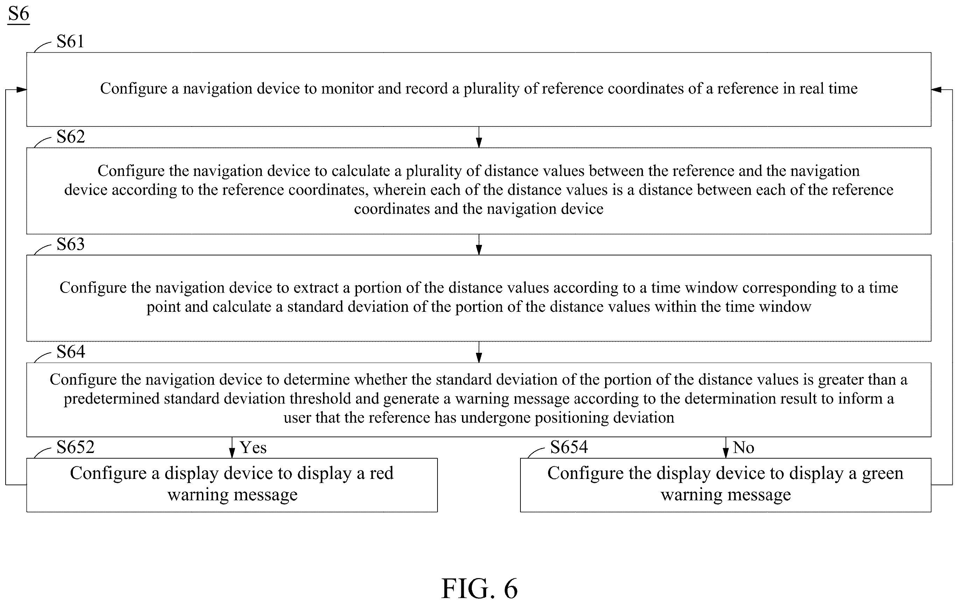

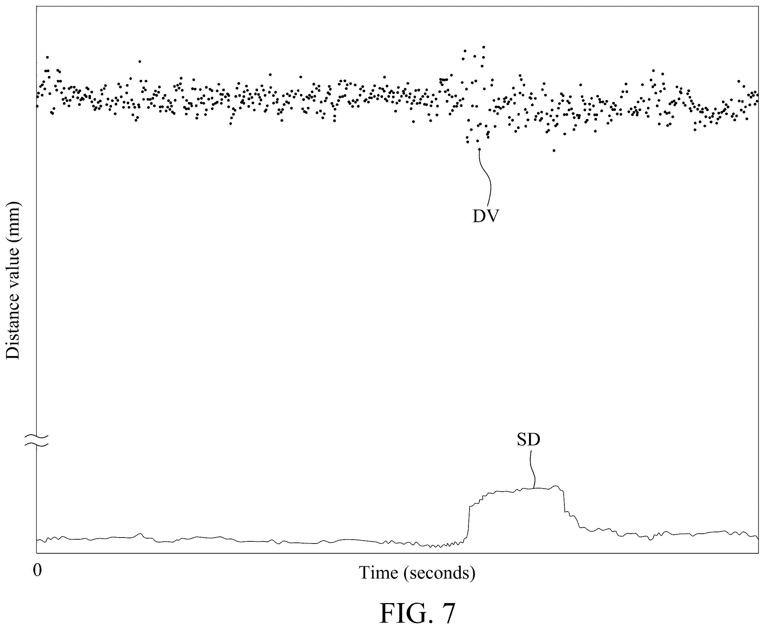

Reference is made to FIGS. 1, 6, and 7 collectively, where FIG. 6 is a flowchart illustrating a positioning deviation warning method S6 for surgical navigation according to a fifth embodiment of the present disclosure; and FIG. 7 is a waveform diagram illustrating motion detection for a dynamic reference 500 according to the present disclosure. The positioning deviation warning method S6 for surgical navigation in FIG. 6 is applied to the positioning deviation warning system 100 for surgical navigation, and includes performing steps S61, S62, S63, S64, S652, and S654. In FIG. 7, the horizontal axis represents time (seconds), and the vertical axis represents distance value (mm).

Step S61 includes configuring a navigation device 300 to monitor and record a plurality of reference coordinates of a reference in real time. In the present embodiment, the reference (reference module) may be a dynamic reference 500 disposed on a second target object T2 (e.g., a C-arm). Step S62 includes configuring the navigation device 300 to calculate and record a plurality of distance values DV between the reference and the navigation device 300 according to the reference coordinates, where each of the distance values DV is a distance between each of the reference coordinates and the navigation device 300. Step S63 includes configuring the navigation device 300 to extract a portion of the distance values DV according to a time window corresponding to a time point and calculate a standard deviation SD of the portion of the distance values DV within the time window. Step S64 includes configuring the navigation device 300 to determine whether the standard deviation SD of the portion of the distance values DV is greater than a predetermined standard deviation threshold to generate a determination result, and generate a warning message according to the determination result to inform a user that the reference has undergone positioning deviation. When the determination result is yes, step S652 is performed; and when the determination result is no, step S654 is performed. Step S652 includes configuring a display device 400 to display a red warning message (indicating that the dynamic reference 500 is unstable). Step S654 includes configuring the display device 400 to display a green warning message (indicating that the dynamic reference 500 is stable).

Specifically, the time window is a window from the time point forward by a time value, and the time value is greater than 2 seconds and less than 4 seconds. The predetermined standard deviation threshold is greater than 0.01 mm and less than 0.05 mm. In one embodiment, the time value may be 3 seconds, and the predetermined standard deviation threshold may be 0.03 mm, but the present disclosure is not limited thereto.

Accordingly, in the positioning deviation warning method S6 for surgical navigation according to the present disclosure, by determining the standard deviation SD of the distance values to confirm whether the reference module has undergone positioning deviation under the condition that the navigation device 300 does not undergo positioning deviation, the degree of stability of the reference module can be displayed in real time, objective judgment criteria can be provided, and the optimal imaging timing can be prompted. Furthermore, according to the present disclosure, a warning message of a specific color is utilized to allow the user to immediately learn of an abnormal displacement (non-periodic displacement), thereby achieving positioning deviation elimination and enhancing surgical accuracy and safety.

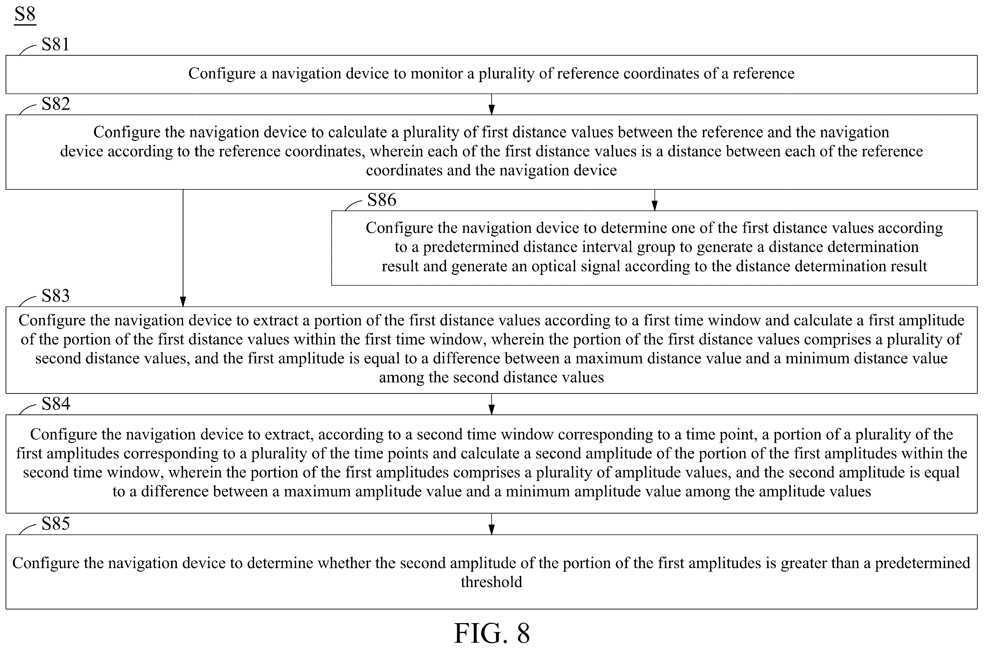

Reference is made to FIGS. 1, 2, 4, and 8 collectively, where FIG. 8 is a flowchart illustrating a positioning deviation warning method S8 for surgical navigation according to a sixth embodiment of the present disclosure. The positioning deviation warning method S8 for surgical navigation is applied to the positioning deviation warning system 100 for surgical navigation, and includes performing steps S81, S82, S83, S84, S85, and S86. Step S81 includes configuring a navigation device 300 to monitor a plurality of reference coordinates of a reference 200. Step S82 includes configuring the navigation device 300 to calculate a plurality of first distance values DV1 between the reference 200 and the navigation device 300 according to the reference coordinates, where each of the first distance values DV1 is a distance between each of the reference coordinates and the navigation device 300. Step S83 includes configuring the navigation device 300 to extract a portion of the first distance values DV1 according to a first time window TW1 and calculate a first amplitude AM1 of the portion of the first distance values DV1 within the first time window TW1, where the portion of the first distance values DV1 includes a plurality of second distance values DV2, and the first amplitude AM1 is equal to a difference between a maximum distance value and a minimum distance value among the second distance values DV2. Step S84 includes configuring the navigation device 300 to extract, according to a second time window TW2 corresponding to a time point TP, a portion of a plurality of the first amplitudes AM1 corresponding to a plurality of the time points TP and calculate a second amplitude AM2 of the portion of the first amplitudes AM1 within the second time window TW2, where the portion of the first amplitudes AM1 includes a plurality of amplitude values, and the second amplitude AM2 is equal to a difference between a maximum amplitude value and a minimum amplitude value among the amplitude values. Step S85 includes configuring the navigation device 300 to determine whether the second amplitude AM2 of the portion of the first amplitudes AM1 is greater than a predetermined threshold.

Furthermore, step S86 is performed after step S82, and includes configuring the navigation device 300 to determine one of the first distance values DV1 according to a predetermined distance interval set to generate a distance determination result, and generate an optical signal according to the distance determination result. The predetermined distance interval set includes a first predetermined distance threshold and a second predetermined distance threshold, and the first predetermined distance threshold is less than the second predetermined distance threshold. The optical signal has a color, and the color is one of a green, a yellow, and an orange. When the distance determination result is that the one of the first distance values DV1 is less than the first predetermined distance threshold, the color of the optical signal is the green. When the distance determination result is that the one of the first distance values DV1 is greater than or equal to the first predetermined distance threshold and less than the second predetermined distance threshold, the color of the optical signal is the yellow. When the distance determination result is that the one of the first distance values DV1 is greater than the second predetermined distance threshold, the color of the optical signal is the orange. In one embodiment, the first predetermined distance threshold is 1.6 m, the second predetermined distance threshold is 1.8 m, and the predetermined distance interval set is (1.6 m, 1.8 m), but the present disclosure is not limited thereto.

Accordingly, in the positioning deviation warning method S8 for surgical navigation according to the present disclosure, by determining the second amplitude AM2 of the first amplitudes AM1 of the distance values (i.e., an amplitude of amplitudes of the distance values) to confirm whether the reference 200 has undergone positioning deviation under the condition that the navigation device 300 does not undergo positioning deviation, not only real-time detection be effectively achieved, but also non-periodic and periodic displacements can be effectively distinguished, thereby achieving the effect of positioning deviation warning. Furthermore, according to the present disclosure, an optical signal of a specific color is utilized to provide an objective distance status and assist the user in maintaining the optimal performance of a system (keeping the system with a green optical signal), so as to ensure the positioning system is in an optimal working state.

Additionally, it is worth mentioning that the positioning deviation warning system 100 for surgical navigation and the positioning deviation warning methods S0, S2, S4, S6, and S8 for surgical navigation according to the present disclosure may also confirm whether the navigation device 300 has undergone positioning deviation under the condition that the reference 200 does not undergo positioning deviation. The reason is that the optical tracker of the navigation device 300 and the display device 400 are mounted on the same frame. If the display device 400 is a touch screen and the user applies excessive force while touching the screen, it may cause the optical tracker to undergo motion or positioning deviation. Therefore, the system and method of the present disclosure can also detect that the navigation device 300 has undergone positioning deviation.

In addition to the motion of the patient and the surgical instrument (e.g., C-arm), other factors causing positioning deviation include: (1) the reference 200 on the surgical instrument or patient is placed at the boundary of the effective detection range of the optical tracker; and (2) the reference 200 on the surgical instrument or patient is damaged, such as scratches or blood stains. The above factors can also cause the amplitude of the amplitudes of the distance values (Reference) or the standard deviation of the distance values (C-arm) to exceed the threshold, and the present disclosure can effectively detect these situations.

Reference is made to FIGS. 3 and 6 collectively. As shown, the methods (i.e., software) of the present disclosure perform a loop to continuously monitor positional changes of the reference 200 or the dynamic reference 500. In FIG. 3, in the positioning deviation warning method S2 for surgical navigation, steps S21, S22, S23, S24, S25, and either step S262 or S264 are continuously repeated. In FIG. 6, in the positioning deviation warning method S6 for surgical navigation, steps S61, S62, S63, S64, and either step S652 or S654 are continuously repeated.

In other embodiments, in addition to determining the stability of an object based on an amplitude of amplitudes of distance values or a standard deviation of the distance values corresponding to the object within the time window, the present disclosure may further combine other dynamic features for discrimination, such as velocity information, acceleration information, or multi-parameter comprehensive judgment. Regarding the velocity information, a tracking system can calculate positional variations of the object at consecutive time points, thereby deriving the instantaneous velocity or average velocity. If there is significant velocity fluctuation, such as frequent velocity changes within a short time, it may indicate that the object is in a motion state. Regarding the acceleration information, since acceleration reflects the rate of velocity change, it has high sensitivity to detecting sudden or intermittent motions. If there is a drastic change in acceleration, it indicates that the object has been subjected to an instantaneous external force or vibration interference. Regarding the multi-parameter comprehensive judgment, indicators such as the amplitude of the amplitudes of the distance values, the standard deviation of the distance values, the velocity information, and the acceleration information can be combined for evaluation to enhance the sensitivity and accuracy of detecting motions.

The above embodiments can be implemented using computer programming products which may include a computer-readable storage medium storing a plurality of instructions that can program a computer to perform the steps in the above embodiments. In other words, the computer-readable storage medium has a plurality of instructions that, when executed by a processor, cause the processor to perform the positioning deviation warning methods S0, S2, S4, S6, and S8 for surgical navigation. The computer-readable storage medium may include, but is not limited to, a floppy disk, an optical disk, a compact disc read-only memory, a magneto-optical disk, a read-only memory, a random access memory, an erasable programmable read-only memory (EPROM), an electronically erasable programmable read-only memory (EEPROM), an optical card or a magnetic card, a flash memory, or any other computer-readable storage medium suitable for storing electronic instructions.

As can be seen from the above implementations, the present disclosure has the following advantages: First, by determining the second amplitude of the first amplitudes of the distance values (i.e., the amplitude of the amplitudes of the distance values) to confirm whether the reference has undergone positioning deviation, not only can real-time detection be effectively achieved, but also non-periodic and periodic displacements can be effectively distinguished, thereby achieving the effect of positioning deviation warning. Second, by using the warning message of a specific color, the user can immediately learn of abnormal displacements (non-periodic displacements), thereby achieving deviation elimination and enhancing surgical accuracy and safety. Third, by determining the standard deviation of the distance values to confirm whether the reference module has undergone positioning deviation, the degree of stability of the reference module can be displayed in real time, objective judgment criteria can be provided, and the optimal imaging timing can be prompted, thereby enhancing the image positioning accuracy, optimizing the surgical workflow efficiency, reducing the uncertainty of subjective judgment by the operator, and improving the overall system reliability. Fourth, by using the optical signal of a specific color to provide an objective distance status and assist the user in maintaining the optimal performance of the system (keeping the system with a green optical signal), the positioning system is ensured to be in an optimal working state.

## Claims

1. A positioning deviation warning method (S0, S2, S8) for surgical navigation, **characterized in** comprising:
configuring a navigation device (300) to monitor a plurality of reference coordinates of a reference (200);
configuring the navigation device (300) to calculate a plurality of first distance values (DV1) between the reference (200) and the navigation device (300) according to the reference coordinates, wherein each of the first distance values (DV1) is a distance between each of the reference coordinates and the navigation device (300);
configuring the navigation device (300) to extract a portion of the first distance values (DV1) according to a first time window (TW1) and calculate a first amplitude (AM1) of the portion of the first distance values (DV1) within the first time window (TW1), wherein the portion of the first distance values (DV1) comprises a plurality of second distance values (DV2), and the first amplitude (AM1) is equal to a difference between a maximum distance value and a minimum distance value among the second distance values (DV2);
configuring the navigation device (300) to extract, according to a second time window (TW2) corresponding to a time point (TP), a portion of a plurality of the first amplitudes (AM1) corresponding to a plurality of the time points (TP) and calculate a second amplitude (AM2) of the portion of the first amplitudes (AM1) within the second time window (TW2), wherein the portion of the first amplitudes (AM1) comprises a plurality of amplitude values, and the second amplitude (AM2) is equal to a difference between a maximum amplitude value and a minimum amplitude value among the amplitude values; and
configuring the navigation device (300) to determine whether the second amplitude (AM2) of the portion of the first amplitudes (AM1) is greater than a predetermined threshold.

2. The positioning deviation warning method (S0, S2, S8) for surgical navigation according to claim 1, wherein the navigation device (300) determines whether the second amplitude (AM2) is greater than the predetermined threshold to generate a determination result, and generates a warning message according to the determination result to inform a user that the reference (200) has undergone positioning deviation.

3. The positioning deviation warning method (S0, S2, S8) for surgical navigation according to any of claims 1-2, wherein the first time window (TW1) is greater than the second time window (TW2).

4. The positioning deviation warning method (S0, S2, S8) for surgical navigation according to any of claims 1-3, wherein,
the first time window (TW1) is a window from the time point (TP) forward by a first time value; and
the second time window (TW2) is a window from the time point (TP) forward by a second time value.

5. The positioning deviation warning method (S0, S2, S8) for surgical navigation according to any of claims 1-4, further comprising:
configuring the navigation device (300) to determine one of the first distance values (DV1) according to a predetermined distance interval set to generate a distance determination result, and generate an optical signal according to the distance determination result;
wherein the predetermined distance interval set comprises a first predetermined distance threshold and a second predetermined distance threshold, the first predetermined distance threshold is less than the second predetermined distance threshold, the optical signal has a color, and the color is one of a green, a yellow, and an orange.

6. The positioning deviation warning method (S0, S2, S8) for surgical navigation according to any of claims 1-5, wherein,
when the distance determination result is that the one of the first distance values (DV1) is less than the first predetermined distance threshold, the color of the optical signal is the green;
when the distance determination result is that the one of the first distance values (DV1) is greater than or equal to the first predetermined distance threshold and less than the second predetermined distance threshold, the color of the optical signal is the yellow; and
when the distance determination result is that the one of the first distance values (DV1) is greater than the second predetermined distance threshold, the color of the optical signal is the orange.

7. The positioning deviation warning method (S0, S2, S8) for surgical navigation according to any of claims 1-6, further comprising:
configuring the navigation device (300) to monitor a plurality of dynamic reference coordinates of a dynamic reference (500);
configuring the navigation device (300) to calculate a plurality of dynamic distance values between the dynamic reference (500) and the navigation device (300) according to the dynamic reference coordinates, wherein each of the dynamic distance values is a distance between each of the dynamic reference coordinates and the navigation device (300);
configuring the navigation device (300) to extract a portion of the dynamic distance values according to a time window and calculate a standard deviation (SD) of the portion of the dynamic distance values within the time window; and
configuring the navigation device (300) to determine whether the standard deviation (SD) of the portion of the dynamic distance values is greater than another predetermined threshold.

8. The positioning deviation warning method (S0, S2, S8) for surgical navigation according to any of claims 1-7, wherein the reference (200) is disposed on a first target object (T1), the dynamic reference (500) is disposed on a second target object (T2), and the first target object (T1) is different from the second target object (T2).

9. A positioning deviation warning method (S4, S6) for surgical navigation, **characterized in** comprising:
configuring a navigation device (300) to monitor a plurality of reference coordinates of a reference (200);
configuring the navigation device (300) to calculate a plurality of distance values (DV) between the reference (200) and the navigation device (300) according to the reference coordinates, wherein each of the distance values (DV) is a distance between each of the reference coordinates and the navigation device (300);
configuring the navigation device (300) to extract a portion of the distance values according to a time window and calculate a standard deviation (SD) of the portion of the distance values within the time window; and
configuring the navigation device (300) to determine whether the standard deviation (SD) of the portion of the distance values is greater than a predetermined standard deviation threshold.

10. The positioning deviation warning method (S4, S6) for surgical navigation according to claim 9, wherein the navigation device (300) determines whether the standard deviation (SD) is greater than the predetermined standard deviation threshold to generate a determination result, and generates a warning message according to the determination result to inform a user that the reference (200) has undergone positioning deviation.

11. The positioning deviation warning method (S4, S6) for surgical navigation according to any of claims 9-10, wherein the time window is a window from a time point (TP) forward by a time value.

12. A positioning deviation warning system (100) for surgical navigation, **characterized in** comprising:
a reference (200); and
a navigation device (300), disposed toward the reference (200) and configured to perform operations comprising:
monitoring a plurality of reference coordinates of the reference (200);
calculating a plurality of first distance values (DV1) between the reference (200) and the navigation device (300) according to the reference coordinates, wherein each of the first distance values (DV1) is a distance between each of the reference coordinates and the navigation device (300);
extracting a portion of the first distance values (DV1) according to a first time window (TW1) and calculating a first amplitude (AM1) of the portion of the first distance values (DV1) within the first time window (TW1), wherein the portion of the first distance values (DV1) comprises a plurality of second distance values (DV2), and the first amplitude (AM1) is equal to a difference between a maximum distance value and a minimum distance value among the second distance values (DV2);
extracting, according to a second time window (TW2) corresponding to a time point (TP), a portion of a plurality of the first amplitudes (AM1) corresponding to a plurality of the time points (TP) and calculating a second amplitude (AM2) of the portion of the first amplitudes (AM1) within the second time window (TW2), wherein the portion of the first amplitudes (AM1) comprises a plurality of amplitude values, and the second amplitude (AM2) is equal to a difference between a maximum amplitude value and a minimum amplitude value among the amplitude values; and
determining whether the second amplitude (AM2) of the portion of the first amplitudes (AM1) is greater than a predetermined threshold.

13. The positioning deviation warning system (100) for surgical navigation according to claim 12, further comprising:
a display device (400), connected to the navigation device (300) and configured to display a warning message;
wherein the navigation device (300) determines whether the second amplitude (AM2) is greater than the predetermined threshold to generate a determination result, and generates the warning message according to the determination result to inform a user that the reference (200) has undergone positioning deviation.

14. The positioning deviation warning system (100) for surgical navigation according to any of claims 12-13, wherein the first time window (TW1) is greater than the second time window (TW2).

15. The positioning deviation warning system (100) for surgical navigation according to any of claims 12-14, wherein,
the first time window (TW1) is a window from the time point (TP) forward by a first time value; and
the second time window (TW2) is a window from the time point (TP) forward by a second time value.

16. The positioning deviation warning system (100) for surgical navigation according to any of claims 12-15, wherein the navigation device (300) is configured to perform operations further comprising:
configuring the navigation device (300) to determine one of the first distance values (DV1) according to a predetermined distance interval set to generate a distance determination result, and generate an optical signal according to the distance determination result;
wherein the predetermined distance interval set comprises a first predetermined distance threshold and a second predetermined distance threshold, the first predetermined distance threshold is less than the second predetermined distance threshold, the optical signal has a color, and the color is one of a green, a yellow, and an orange.

17. The positioning deviation warning system (100) for surgical navigation according to any of claims 12-16, wherein,
when the distance determination result is that the one of the first distance values (DV1) is less than the first predetermined distance threshold, the color of the optical signal is the green;
when the distance determination result is that the one of the first distance values (DV1) is greater than or equal to the first predetermined distance threshold and less than the second predetermined distance threshold, the color of the optical signal is the yellow; and
when the distance determination result is that the one of the first distance values (DV1) is greater than the second predetermined distance threshold, the color of the optical signal is the orange.

18. The positioning deviation warning system (100) for surgical navigation according to any of claims 12-17, wherein the reference (200) is disposed on a first target object (T1), and the positioning deviation warning system (100) for surgical navigation further comprises:
a dynamic reference (500) disposed on a second target object (T2), wherein the first target object (T1) is different from the second target object (T2);
wherein the navigation device (300) is configured to perform operations further comprising:
monitoring a plurality of dynamic reference coordinates of the dynamic reference (500);
calculating a plurality of dynamic distance values between the dynamic reference (500) and the navigation device (300) according to the dynamic reference coordinates, wherein each of the dynamic distance values is a distance between each of the dynamic reference coordinates and the navigation device (300);
extracting a portion of the dynamic distance values according to a time window and calculating a standard deviation (SD) of the portion of the dynamic distance values within the time window; and
determining whether the standard deviation (SD) of the portion of the dynamic distance values is greater than another predetermined threshold.
